# EUROPEAN PATENT APPLICATION

(11) **EP 2 186 822 A1**
(43) Date of publication of application: **19.05.2010**
(21) Application number: 08291041.5
(22) Date of filing: 07.11.2008
(51) Int. Cl.: C07K 14/47, A61K 38/57

(54) **Use of inhibitors of Plac8 activity for the modulation of adipogenesis**

(71) Applicant: Sanofi-Aventis, 75013 Paris (FR)
(72) Inventor: Hall, Diana, 1007 Lausanne (CH); Jimenez, Maria, 1022 Chavannes-près-Renens (CH); Poussin, Carine, 74500 Evian-les-Bains (FR); Thorens, Bernard, 1066 Epalinges (CH)
(74) Representative: Bouvet, Philippe

(57) **Abstract**

The present invention concerns Plac8, a new target involved in adipogenesis modulation. Using a siRNA approach, the inventors demonstrated that decrease in Plac8 activity in preadipocytes and adipose tissue induces a decrease in adipogenesis. Thus, the present invention relates to modulators of Plac8 activity as well as screening test for identification of modulators of the activity of this target, and their use, especially in pharmaceutical composition, to modulate adipogenesis and thus treat obesity and related disorders.

## Description

The present invention concerns Plac8, a new target involved in adipogenesis modulation as well as screening test for identification of modulators of the activity of this target. Further, the present invention relates to modulators of Plac8 activity and their use, especially in pharmaceutical composition, to modulate adipogenesis and thus treat obesity and related disorders.

Obesity is a major risk factor for a number of disorders including hypertension, coronary artery disease, dyslipidemia, insulin resistance and type 2 diabetes. Because of the importance of the obesity epidemic, a great deal of investigation has centered on the biology of the adipocyte, including the developmental pathway by which new adipocytes are created. Adipogenesis is the process by which undifferentiated mesenchymal precursor cells become mature adipocytes. Throughout the last decade considerable progress has been made in elucidating the molecular mechanisms of adipocyte differentiation, which involve sequential activation of transcription factors from several families such as CCAAT/enhancer binding proteins (C/EBPα, α, and γ) and the nuclear hormone receptor peroxisome proliferator-activated receptor γ (PPARγ) (Rosen, E.D. et *al*., 2002). PPARγ is described as a "master regulator" of adipogenesis since it has been shown to be both sufficient and necessary for adipogenesis both *in vitro* and *in vivo.* Recently, new transcription factors have been described to participate in adipogenesis such as KLF family (KLF2, 5 and KLF15) (Banerjee, S. S. et *al*., 2003; Gray, S. M. et *al*., 2002), Ebf family (Jimenez, M. A. et *al*., 2007) and Krox 20 (Chen, Z. et *al*., 2005), suggesting that the transcriptional cascade occurring during adipogenesis is much more complex than previously thought. Furthermore, signaling molecules and/or receptors such as the Wnt family of secreted proteins (Kang S. et *al*., 2007), sonic hedgehog protein, Notch receptor have also been described to be involved in molecular events leading to adipocyte formation. It is interesting to note that extracellular and intracellular events are somehow coupled to regulate adipogenesis. All these signaling pathways converge on a tightly regulated transcriptional cascade, which needs to be more completely understood to potentially control adipocyte development and prevent obesity.

Storage of fat in adipose tissue is limited and exceeding this capacity leads to accumulation of lipids in others tissues, in particular in muscle, liver, and the endocrine pancreas, and to the secretion by adipocytes of various adipokines. The adipose tissue consists of several deposits located at different anatomical sites which may originate from distinct precursors and which have different physiological functions and pathophysiological roles. The visceral, as opposed to the subcutaneous adipose depots, may contribute more to the defects associated with the metabolic syndrome.

Cannabinoid 1 receptors have been identified in all organs playing a key role in glucose metabolism and type 2 diabetes, i.e. adipose tissue, the gastrointestinal tract, the liver, the skeletal muscle and the pancreas. Rimonabant, the first selective cannabinoid receptor 1 (CB1 R) antagonist in clinical use, has been shown to reduce food intake and body weight thus improving glucose metabolism regulation.

However, there is still a need for novel therapeutic targets for the treatment of obesity.

Placental 8 protein (Plac8) is known as a cytoplasmic signaling molecule, although it has been reported to have a putative signal peptide (Rogulski, K. et *al*., 2005). Recently, Plac8 knockout mice were generated and exhibited an impaired immune response to bacteria infection (Ledford, J. G. *et al*., 2007). The role and function of Plac8 in immune cells, as well as in other cell types is still unknown.

The inventors have now found that Plac8 plays a critical role in adipocyte differentiation. Plac8 is thus considered as a new relevant target for modulation of adipogenesis and for the treatment of obesity and related disorders. Inhibition of Plac8 can also be used to decrease adipogenesis for reduction of subcutaneousand visceral fat accumulation.

### Detailed description of the invention

The present invention is drawn to methods for regulating adipogenesis and metabolic function in adipocytes.

The present invention consists in the use of inhibitors of Plac8 activity for modulation of adipogenesis, in particular for treatment of obesity and related disorders. The invention also concerns pharmaceutical composition containing such modulators of adipogenesis and related disorders and screening test for such modulators.

The inventors have identified le role of Plac8 in adipogenesis modulation. Through a transcriptomic approach, they identified genes whose expression was correlated with body weight gain in cohorts of C57BI/6 mice fed a high fat diet. Then, they conducted a second analysis in order to evaluate the changes in gene expression induced by rimonabant treatment of the high fat diet fed mice. Genes which have never been described before in adipocyte biology, but which might be involved in important biological processes such as signaling, modification of extracellular matrix proteins, and gene transcription were retained. These genes could be important for adipogenesis especially since they might be involved in the mechanism by which rimonabant reduces fat mass in mice. In this context, Plac8 was identified as involved in adipocytes metabolism, especially in new signaling pathway. More generally, this gene appears to play a role in adipogenesis and control of adipose tissue development in obesity.

The present invention consists in identification of modulators of Plac8 activity. Such modulators can be any compound or molecule able to modulate Plac8 activity in particular small molecules, lipids and siRNA.

Modulators of Plac8 activity can be identified by detecting the ability of an agent to modulate the activity of Plac8. Inhibitors of Plac8 are any compound able to reduce or inhibit, totally or partially, the activity of Plac8. Inhibitors of Plac8 include, but are not limited to, agents that interfere with the interaction of Plac8 with its natural ligand in the intracellular compartment, agents that reduce Plac8 expression, both at transcriptional and translational levels, as well as agents that inhibit intracellular signals wherein Plac8 is involved.

In one embodiment, Plac8 activity can be reduced using small molecules that inhibit, totally or partially, the transcription of Plac8. Such modulators can be identified using methods well known by the person skilled of the art, as a reporting system consisting in the promoter of Plac8 linked in frame to a reporter gene and expressed in a suitable cell line; the reporter gene product's activity can be quantitatively measured. Thus, a compound that inhibits the expression of the reporter gene, for example by inhibiting an activating transcription factor, can be considered as a potential candidate.

The reporter genes that can be used in such reporting systems are numerous and well known in the art. For example, such reporter genes can be genes allowing expression of Green Fluorescent Protein (GFP), luciferase, β-galactosidase...

Therefore, one aspect of the present invention is to provide a method for screening for inhibitors of the activity of Plac8 which comprises the steps of:
a) transfecting a cell line with a reporter construction comprising a Plac8 promoter linked to a reporter gene
b) cultivating said cell line in condition to allow expression of the reporter gene
c) adding candidate compounds into the cell culture, and
d) identifying inhibitor compounds as being those compounds which have the ability to reduce or inhibit the reporter gene expression

The predicted promoter of Plac8 to be used in the described above screening test for modulators of Plac8 transcription corresponds to SEQ ID NO.23.

In another embodiment, the expression of Plac8 is modulated through RNA interference, using small interfering RNAs (siRNA) or small hairpin RNAs (shRNAs). Therefore, in one aspect, the present invention relates to double stranded nucleic acid molecules including small nucleic acid molecules, such as short interfering nucleic acid (siNA), short interfering RNA (siRNA), double-stranded RNA (dsRNA), micro-RNA (miRNA), and short hairpin RNA (shRNA) molecules able to mediate RNA interference (RNAi) against Plac8 gene expression, including cocktails of such small nucleic acid molecules and suitable formulations of such small nucleic acid molecules.

The phenomenon of RNAi mediated gene silencing has been described first in the Caenorhabditis elegans system, in which microinjection of long double stranded RNA molecules was reported. The mechanism of RNA mediated gene inactivation seems to be slightly different in the various organisms that have been investigated so far. However, in all systems, RNA mediated gene silencing is based on post-transcriptional degradation of the target mRNA induced by the endonuclease Argonaute2 which is part of the so called RISC complex. Sequence specificity of degradation is determined by the nucleotide sequence of the specific antisense RNA strand loaded into the RISC complex.

The introduction into cells of an siRNA compound results in cells having a reduced level of the target mRNA and, thus, of the corresponding polypeptide and, concurrently, of the corresponding enzyme activity. siRNAs specific for Plac8, as described herein, can be used as modulators of Plac8 activity, in order to reduce the translation of Plac8 mRNA. More particularly, siRNA specific for Plac8 can be used to reduce adipogenesis and thus to treat obesity and related diseases.

In one embodiment, the invention features a double stranded nucleic acid molecule, such as a siRNA molecule, where one of the strands comprises nucleotide sequence having complementarity to a predetermined Plac8 nucleotide sequence in a target Plac8 nucleic acid molecule, or a portion thereof.

The RNA molecule can be used modified or unmodified. An example of modification is the incorporation of tricylo-DNA to allow improved serum stability of oligonucleotide.

In one embodiment, the determined Plac8 nucleotide sequence is a Plac8 nucleotide target sequence described herein (SEQ ID NO.1 and SEQ ID NO.3).

Due to the potential for sequence variability of the genome across different organisms or different subjects, selection of siRNA molecules for broad therapeutic applications likely involves the conserved regions of the gene. Thus in one embodiment, the present invention relates to siRNA molecules that target conserved regions of the genome or regions that are conserved across different targets. siRNA molecules designed to target conserved regions of various targets enable efficient inhibition of Plac8 gene expression in diverse patient populations.

In one embodiment, the invention features a double-stranded short interfering nucleic acid molecule that down-regulates expression of a target Plac8 gene or that directs cleavage of a target RNA, wherein said siRNA molecule comprises about 15 to about 28 base pairs, preferably about 19 base pairs. A siRNA or RNAi inhibitor of the instant invention can be chemically synthesized, expressed from a vector or enzymatically synthesized.

In a particular embodiment, the siRNA specific for Plac8 are shRNA having sequence SEQ ID NO.5 or SEQ ID NO.6 or SEQ ID NO.7. In a preferred embodiment, the siRNA specific for Plac8 are shRNA having sequence SEQ ID NO.6 or SEQ ID NO.7 and in a more preferred embodiment, the siRNA specific for Plac8 is shRNA having sequence SEQ ID NO.6.

The use of a siRNA according to the present invention leads to reduction of the mRNA level from 5% to 20%, preferably from 5% to 15%, more preferably from 5% to 10% of the mRNA level of the corresponding wild type cell. The wild type cell is the cell prior to the introduction of the nucleic acid encoding the siRNA compound, in which the targeted mRNA is not degraded by a siRNA compound.

Inhibitors of Plac8 activity can be administrated by any suitable route, both locally or systemically depending on the nature of the molecule and the expected effect. SiRNA can be administrated locally in case of double strand molecule directly in the targeted tissue, or administrated through a vector in case of shRNA, according to protocols used in the art.

In one embodiment, RNAi is obtained using shRNA molecules. ShRNA constructs encode a stem-loop RNA. After introduction into cells, this stem-loop RNA is processed into a double stranded RNA compound, the sequence of which corresponds to the stem of the original RNA molecule. Such double stranded RNA can be prepared according to any method known in the art including vitro and *in vivo* methods as, but not limited to, described in Sahber *et al* (1987), Bhattacharyya *et al,* (1990) or US 5, 795, 715.

For *in vivo* administration, shRNA can be introduced into a plasmid. Plasmid-derived shRNAs present the advantage to provide the option for combination with reporter genes or selection markers, and delivery via viral or non viral vectors. The introduction of shRNA into a vector and then into cells ensure that the shRNA is continuously expressed. The vector is usually passed on to daughter cells, allowing the gene silencing to be inherited.

The present invention also provides vectors comprising the polynucleotides for expression of shRNA expression of the invention. These vectors are for example AAV vector, retroviral vector in particular lentiviral vector, adenoviral vector which can be administered by different suitable routes including intravenous route, intramuscular route, direct injection into subcutaneous tissue or other targeted tissue chosen according to usual practice.

The route of administration of siRNA varies from local, direct delivery to systemic intravenous administration. The advantage of local delivery is that the doses of siRNA required for efficacy are substantially low since the molecules are injected into or near the target tissue. Local administration also allows for focused delivery of siRNA. For such direct delivery, naked siRNA can be used. "Naked siRNA" refers to delivery of siRNA (unmodified or modified) in saline or other simple excipients such as 5% dextrose. The ease of formulation and administration of such molecules makes this an attractive therapeutic approach. Naked DNA can also be formulated into lipids especially liposomes.

Systemic application of siRNA is often less invasive and, more importantly, not limited to tissues which are sufficiently accessible from outside. For systemic delivery, siRNA can be formulated with cholesterol conjugate, liposomes or polymer-based nanoparticules. Liposomes are traditionally used in order to provide increased pharmacokinetics properties and/or decreased toxicity profiles. They allow significant and repeated successful *in vivo* delivery. Currently, use of lipid-based formulations of systemic delivery of siRNA, especially to hepatocytes, appears to represent one of the most promising near-term opportunities for development of RNAi therapeutics. Formulation with polymers such as dynamic polyconjugates - for example coupled to N-acetylglucosamine for hepatocytes targeting - and cyclodextrin-based nanoparticules allow both targeted delivery and endosomal escape mechanisms. Others polymers such as atelocollagen and chitosan allow therapeutic effects on subcutaneous tumor xenografts as well as on bone metastases.

SiRNA can also be directly conjugated with a molecular entity designed to help targeted delivery. Given the nature of the siRNA duplex, the presence of the inactive or sense stand makes for an ideal site for conjugation. Examples of conjugates are lipophilic conjugates such as cholesterol, or aptamer-based conjugates.

Cationic peptides and proteins are also used to form complexes with the negatively charged phosphate backbone of the siRNA duplex.

These different delivery approaches can be used to target the Plac8 siRNA into the relevant tissue, especially adipose tissue. For such targeting, siRNA can be conjugated to different molecules interacting with pre-adipocytes and adipocytes, as for example ligands interacting with lipids transporters, receptors, insulin receptor or any molecule known in the art.

Another object of the invention is a pharmaceutical composition, which comprises, as active principle, a modulator of Plac8 according to the present invention. These pharmaceutical compositions comprise an effective dose of at least one modulator according to the invention, and at least one pharmaceutically acceptable excipient. Said excipients are chosen according to the pharmaceutical form and the administration route desired, among usual excipients known of one of skill in the art.

The invention also consists in a method for modulation of adipogenesis. Such method can be used to treat obesity or related diseases. Such method can also be used in order to decrease fat accumulation in a cosmetic purpose.

Modulators of Plac8 activity are useful in therapeutics to modulate adipogenesis, in particular in the treatment and prevention of obesity related disorders, in particular type 2 diabetes, dyslipidemia, elevated blood pressure, insulin resistance, cardiovascular disorders and more generally metabolic syndromes.

The present invention, according to another of its aspects, relates to a method for the treatment of the above pathologies, which comprises the *in vivo* administration to a patient of an effective dose of a modulator of Plac8 according to the invention.

The appropriate unitary dosage forms comprise the oral forms, such as tablets, hard or soft gelatin capsules, powders, granules and oral solutions or suspensions, the sublingual, buccal, intratracheal, intraocular, intranasal forms, by inhalation, the topical, transdermal, sub-cutaneous, intramuscular or intra-venous forms, the rectal forms and the implants. For the topical application, the compounds of the invention may be used as creams, gels, ointments or lotions.

According to usual practice, the dosage suitable to each patient is determined by the physician according to the administration route, the weight and response of the patient.

Plac8 inhibitors are also useful for cosmetic applications in order to reduce disgraceful fat accumulation. For cosmetic applications, inhibitors of Plac8 can be incorporated in a suitable formulation for topical use. The inhibitors of Plac8 can both be small molecules or siRNA as previously described.

The invention is now described by reference to the following examples, which are illustrative only, and are not intended to limit the present invention.

### Examples

### Brief description of the figures

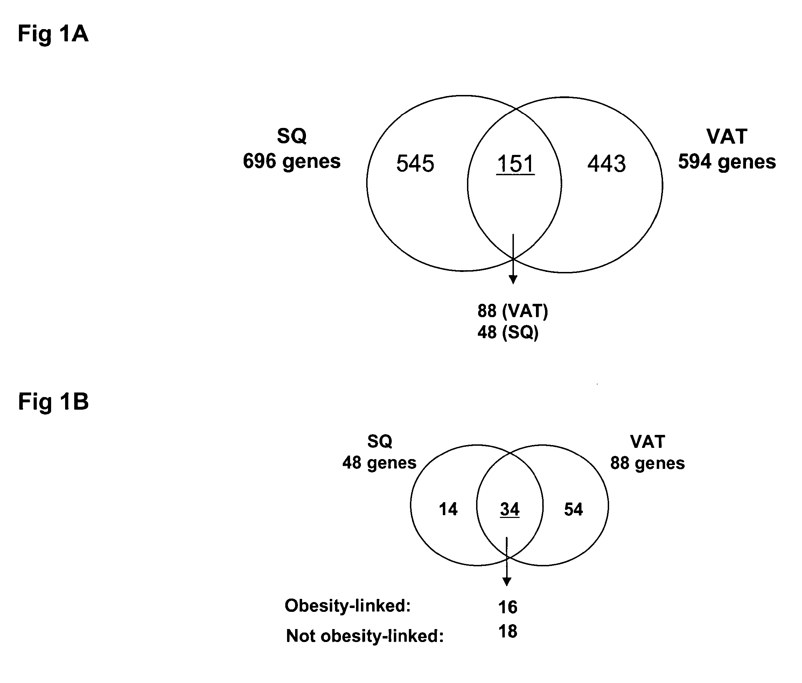

**Figure 1****: Selection of critical adipose tissue regulatory genes.** The Venn diagrams illustrate the selection of genes based on the following criteria. **A**) Similar regulation by high fat feeding in subcutaneous (SCAT or Sq) and visceral (VAT). 151 genes were selected (48 for SCAT and 88 for VAT). **B**) Among those 151 genes, selection of genes regulated by rimonabant treatment (14 for SCAT and 54 for VAT). This led to the selection of 34 genes regulated in both tissues by high fat feeding and rimonabant. Among those genes, 16 have expression level correlated with body weight of L, M and H groups (obesity-linked) and 18 are regulated by HFD to the same level in each subgroup (not obesity-linked).

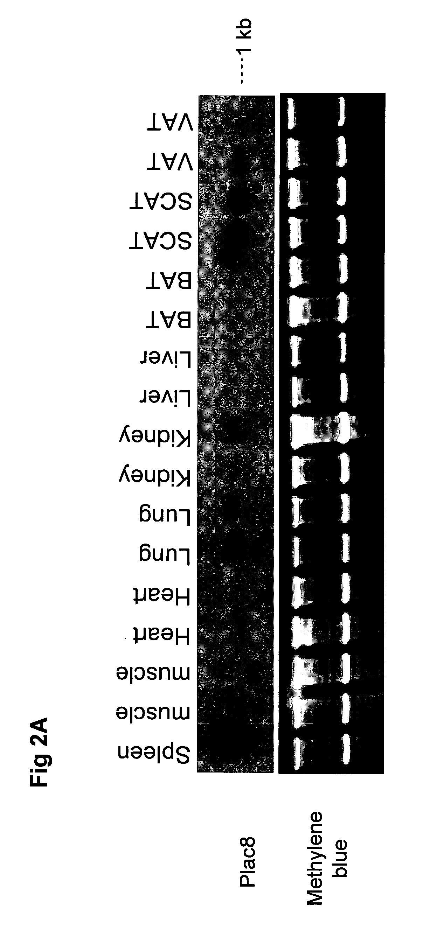

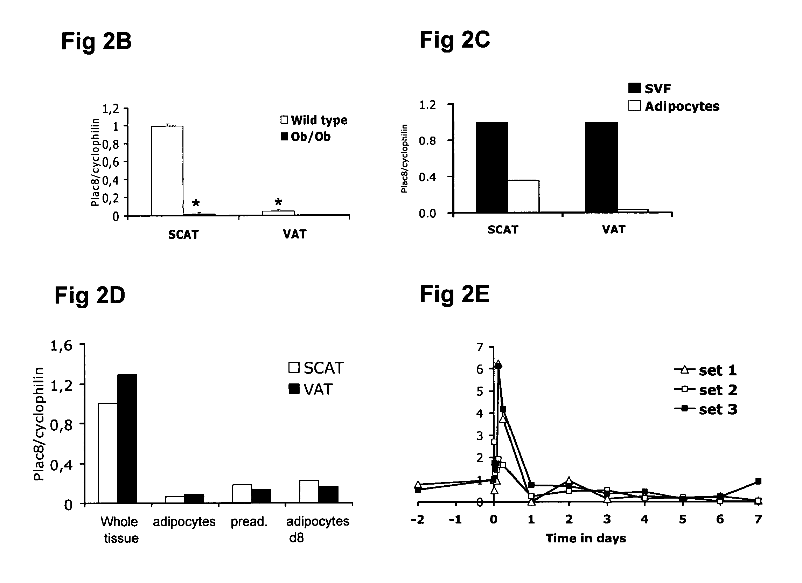

**Figure 2****: Plac8 expression in various tissue and cell types A)** Northern Blotting for Plac8 showing mRNA expression in various mouse tissues: spleen, muscle (gastrocenemius), heart, lung, kidney, liver, brown adipose tissue (BAT), subcutaneous (SCAT) and visceral (VAT) adipose tissues. As a control the membrane is stained with methylene blue. The size of Plac8 mRNA is shown on the right. **B to E:** mRNA levels of Plac8 measured by RT-PCR **B**) in SCAT and VAT of wild-type and Ob/Ob mice (n=5) ^{*} p<0.05, data are shown as mean ± sd and expressed as fold increase relative to the control SCAT set at 1. **C**) in stromal vascular fraction (SVF) and isolated adipocytes of mice (n=5 mice pooled for each extraction, experiment was repeated 3 times, a representative experiment is shown). Data are expressed as fold increase relative to SCAT SVF expression. **D**) in human whole tissue SCAT and VAT, isolated adipocytes, isolated preadipocytes and adipocytes differentiated *in vitro.* Data are expressed as levels relative to whole tissue SCAT expression set arbitrary at 1. **E**) in 3T3-L1 cells prior DMI treatment day-2 and after DMI treatment until day 7. N=2-3 sets of cells. Data are represented as levels relative to the expression at day 0.

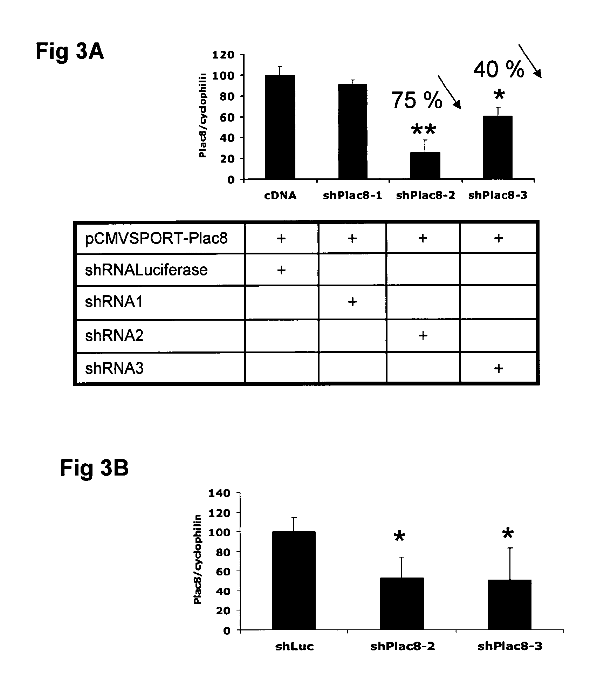

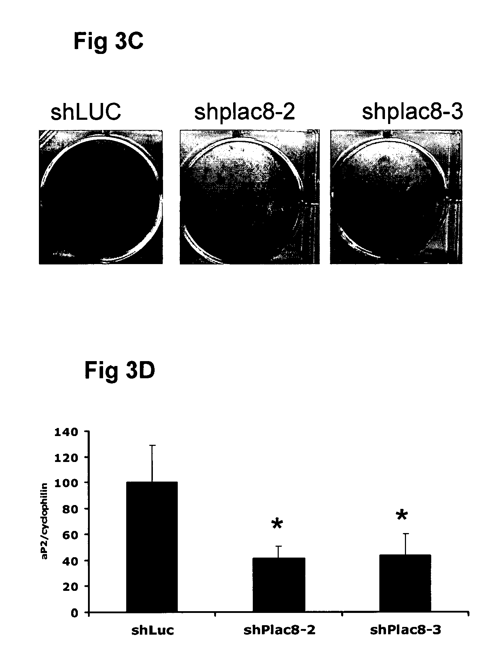

**Figure 3****: Knockdown of Plac8 expression and activity by shRNA A)** shRNA transfection into 293T cells. pSIREN retroviral plasmids containing shRNA sequences against Plac8 were co-transfected with pCMVSPORT expressing plasmid. As a control for shRNA construct, we used a shRNA against the firefly luciferase protein (shRNA luciferase). 3 shRNA were tested for Plac8. **B**) 3T3-L1 cells were transduced with retroviruses containing shRNA directed against luciferase (shLuc) or Plac8 (shPlac8). mRNA levels were measured by RT-PCR prior differentiation. **C**) Oil-red-O pictures of differentiated 3T3-L1 at day 9. **D**) aP2 (marker of differentiation) mRNA expression measured by RT-PCR in the same cells as in C) at day 9. Results are expressed as mean ± sd *P<0.05, **, P<0.01; ***, P<0.005. n=3.

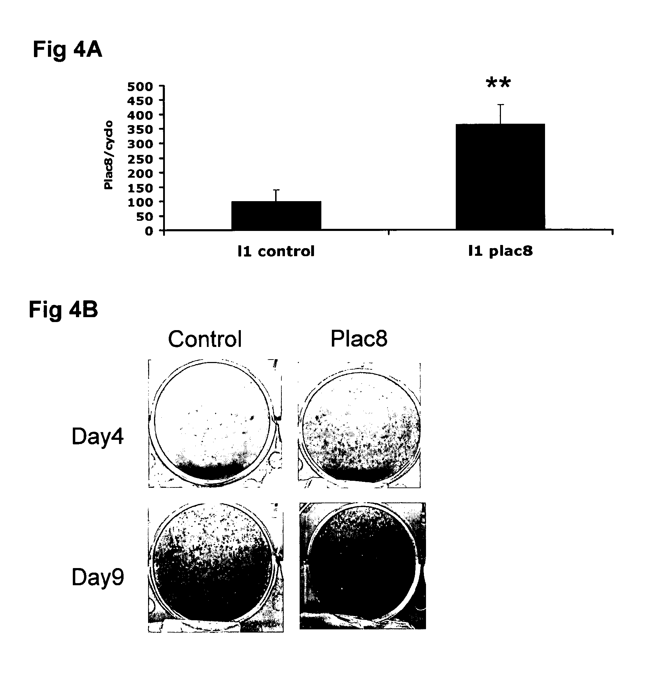

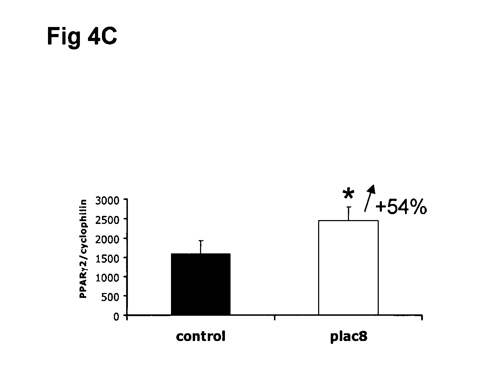

**Figure 4****: Overexpression of Plac8 cDNA in 3T3-L1 cell line A)** 3T3-L1 transduced with retroviruses expressing the murine cDNA for Plac8 or the empty retroviruses as a control. Plac8 mRNA expression measured by RT-PCR at day 0. **B**) Oil-red-O pictures of the dishes of differentiated 3T3-L1 at day 4 and 9 transduced either with construct containing cDNA for Plac8 or empty construct retroviruses (control). **C**) PPARgamma2 (marker of differentiation) mRNA expression measured by RT-PCR in the same cells at day 9. Results are expressed as mean±sd *P<0.05, **, P<0.01. n=3.

### Material and Methods

### Animals treatment

C57BL/6J mice, which are obesity-prone (Collins et *al*. 2004), were fed for 6 months with a high fat diet (HFD). After 6 months of HFD, mice exhibited scattered body weights with various degrees of glucose intolerance (measured by a glucose tolerance test. The HFD mice were separated into 3 groups displaying the same level of glucose intolerance but with low (L), medium (M) or high (H) body weights and treated them, as well as normal chow (NC) fed mice, for one month with vehicle or rimonabant (10 mg.kg⁻¹.day⁻¹), to normalize their body weight.

### RNA preparation, labeling and hybridization on cDNA microarrays.

RNA from 5 different mice per group was extracted from visceral and subcutaneous adipose tissues using peqGOLD Trifast^{™} (peqlab) and chloroform-isoamylalcool (24:1) extraction. RNA was precipitated with isopropanol and purified by passage over RNeasy columns (Qiagen). RNA quality was checked before and after amplification with a Bioanalyzer 2100 (Agilent). RNA was reverse transcribed and RNA was amplified with MessageAmp^{™} kit (Ambion). A Mouse Universal Reference (Clontech) was similarly amplified and both adipose tissue and reference RNAs were labeled by an indirect technique with Cy5 and Cy3 according to published protocols (De Fourmestraux et al., 2004). Labeled RNAs were hybridized to microarrays containing 17664 cDNAs prepared at the DNA Array Facility of the University of Lausanne. Scanning, image, and quality control analyses were performed as previously published (de Fourmestraux et al., J. Biol. Chem. 2004 279:50743-53). Data were expressed as log₂ intensity ratios (Cy5/Cy3), normalized with a print tip locally weighted linear regression (Lowess) method and filtered based on spot quality and incomplete annotation. All analyses were performed with the R software for statistical computing available at the Comprehensive R Archive Network (cran.us.r-project.org/).

### Cell culture

3T3-L1 cells were cultured in DMEM (Gibco) with 10 % FBS (Gibco) at 5 % CO₂. After retroviral infection (see below), cells were allow to grow to confluence in either 100-mm or 60-mm dishes in DMEM with 10 % FBS. Once confluence was reached, cells were exposed to differentiation medium containing dexamethasone (1µM), insulin (5 µg/ml), and isobutylmethylxanthine (0.5 µM) (DMI). After 2 days cells were maintained in medium containing insulin (5 µg/ml) until ready for harvest at 7 days.

### Oil-red-O staining

After 7 to 10 days of differentiation, cells were washed once in PBS and fixed with formaldehyde (Formalde-fresh; Fisher) for 15 minutes. The staining solution was prepared by dissolving 0.5 g oil-red-O in 100 ml of isopropanol; 60 ml of this solution was mixed with 40 ml of distilled water. After 1 hour at room temperature the staining solution was filtered and added to dishes for 4 hours. The staining solution was then removed and cells were washed twice with distilled water.

### shRNA constructs

shRNAs were constructed using the RNAi-Ready pSIREN-RetroQ ZsGreen (Clontech). Target sequences for Plac8 were designed by querying the Whitehead siRNA algorithm (http://jura.wi.mit.edu/bioc/siRNAext/) as well as the siRNA designer software from Clontech (http://bioinfo.clontech.com/rnaidesigner/); at least two sequences represented by both algorithms were subcloned into the pSIREN vectors (Clontech) using the EcoRI and BamH1 restriction sites. The three following target sequences for Plac8 were chosen: SEQ ID NO. 5 (shPlacB-1), SEQ ID NO.6 (shPlac8-2) and SEQ ID NO.7 (shPlac8-3); As a negative control, a siRNA sequence against luciferase having sequence SEQ ID NO. 8 (shLuc) was used.

### Transfection of shRNA constructs

The specificity of shRNAs was tested in 293T HEK cells co-transfected using calcium-Phosphate methods described in Jordan, M., et al. (2004) with expression vectors containing Plac8 cDNA (SEQ ID NO.21) and the RNAi-Ready pSIREN-RetroQ ZsGreen vector expressing either the shRNA against lucifeare (control shLUC) or Plac8 (shPlac8). RT-PCR analysis was performed on cell RNA-extraction 24h after transfection.

### Generation of retroviral constructs and retroviral infections

Retroviruses were constructed in the RNAi-Ready pSIREN-RetroQ ZsGreen (pSIREN Clontech) or pMSCV puromycin plasmid (pMSCV, Clontech). Viral constructs were transfected using calcium-phosphate method described in Jordan, M., *et al.* (2004) into 293 HEK packaging cells along with constructs encoding gag-pol and the VSV-G protein. Supernatants were harvested after 48h in presence of 3µm of Trichostatin A (Sigma) and either used immediately or snap frozen and stored at -80°C for later use. Viral supernatants were added to the cells for 6 hours in the presence of polybrene (4 µg/ml) and diluted two times with fresh medium for the next 15 hours.

### Overexpression constructs

A modified pMSCV puromycin retroviral plasmid (from Clontech) expressing a GFP marker was used to over-expressed the cDNA of Plac8 into cells. The cDNA (SEQ ID NO.21) was inserted blunted into the hpal restriction site from the multicloning site of pMSCV. The resulting colonies were tested for the right orientation and selected by enzymes digestion. The right clone was selected and amplified and used for retroviral infection of 3T3-L1 cells.

### Isolation of adipocytes and stromal vascular fraction (SVF) from adipose tissue

Eights week-old male C57BL/6J mice (n=6-8) were euthanized by CO₂ inhalation and epididymal (visceral) and subcutaneous adipose tissue were collected and placed in DMEM medium containing 10mg/mL fatty acid-poor BSA (Sigma-Aldrich, St. Louis, MI). The tissue was minced into fine pieces and then digested in 0.12 units/mL collagenase type I (Sigma) at 37°C in a shaking water bath (80Hz) for 1 hour. Samples were then filtered through a sterile 250µm nylon mesh (Scrynel NY250HC, Milian) to remove undigested fragments. The resulting suspension was centrifuged at 1100 RPM for 10 min to separate SVF from adipocytes. Adipocytes were removed and washed with DMEM buffer. They were then suspended in peqGOLD TriFast reagent (Axonlab) and RNA was isolated according to the manufacturer's instructions. The SVF fraction was incubated in erythrocyte lysis buffer (0.154mM NH₄CI, 10mM KHCO₃, 0.1mM EDTA) for 2 min. Cells were then centrifuged at 1100 RPM for 10 min and resuspended in 500 µl of peqGOLD TriFast reagent (Axonlab) for RNA isolation.

### RNA extraction and Real-time PCR

Total RNA was isolated from cultured cells using peqGOLD TriFast reagent according to the manufacturer's instructions (Axonlab). First strand cDNA was synthesized from 0.5 µg of total RNA using random primers and Superscript II (Invitrogen). Real time PCR was performed using Power SYBR Green Mix (Applied Biosystem). The following primers were used for mouse genes: SEQ ID NO.9 (Plac8-Forward), SEQ ID NO.10 (Plac8-Reverse), SEQ ID NO.11 (PPARgamma2-F), SEQ ID NO.12 (PPPARgamma2-R), SEQ ID NO.13 (Ap2-F), SEQ ID NO.14 (Ap2-R), SEQ ID NO.15 (Cyclophilin A-F) SEQ ID NO.16 (Cyclophilin A-R). The following primers were used for human genes: SEQ ID NO.17 (hPlac8-F), SEQ ID NO.18 (hPlac8-R), SEQ ID NO.19 (hCyclophilin A-F) and SEQ ID NO.20 (hCyclophilin A-R).

### Northern Blot

Total RNA from various mouse tissues was isolated using the peqGOLD TriFast reagent according to the manufacturer's instructions (Axonlab). Total RNA (8 µg) was separated on a 1,2 % agarose/forlmaldehyde gel and transfected overnight to a nylon membrane. To control for RNA quantity loading, the membrane was stained with methylene blue prior the subsequent hybridizations. For the detection of Plac8 signals, probes from the full-length cDNA mouse plasmid (Open Biosystem) were used. The probes were labeled by random priming with [α-³²P]dCTP (Amersham). Hybridization and washing were carried out using the Quickhib method according to manufacturer's instructions (Stratagene). Blots were exposed to Hyperfilm ECL (Amersham) at -80°C for 1 day or several days depending on the signal intensity.

### Results

### Example 1: Microarray results

Bioinformatic analysis of the microarray data was performed to identify genes that fulfilled the three following criteria: (i) regulated by high fat feeding, (ii) similar regulated expression by high fat feeding in both visceral and subcutaneous fat and (iii) similar normalization of their expression by Rimonabant treatment (Figure 1). Out of the ∼17'000 gene targets present on the cDNA microarray used, 34 genes fulfilled these criteria, which are listed in Table 1. Remarkably, 10 of these genes - Cav1, Fgf1, Fndc3b, Kif5b, Mest, Npr3, Pik3ca, Sparc, Vldlr, and Wwtr1 - were previously known to be important regulators of adipose tissue development and function. Some of these genes had expression levels correlated with body weight gain (shown in grey in Table 1), suggesting a potential role in hyperplasia and/or hypertrophy of adipose tissues during obesity. These results validate the approach used to identify possible novel targets for therapeutic treatment of obesity.

Most importantly, many of the genes cited in table 1 have never been studied in the context of in adipose tissue development or biology. These genes belong to the following classes of function: extracellular matrix/cell interaction, cytoskeleton, intracellular signaling, enzymes, and transcription factors/co-factors. They are likely involved in tissue remodeling, and particularly in adipocyte development. One of these genes, Plac8 gene and its role in adipocyte biology, is presented herein and constitutes one aspect of the present invention.

The mouse and human sequences of Plac8 as used in the present invention corresponds to SEQ ID NO.1 and SEQ ID NO.3 respectively.

**Table 1: List of 34 gene candidates regulated by HFD and rimonabant in SCAT and VAT. The full name and gene symbol are showed in the first column. The biological role for known genes and references are indicated in the second column. All these genes were up-regulated by HFD and normalized by rimonabant treatment, excepted for Plac8 and Rp9h, which were down-regulated by HFD. The genes correlated to body weight increase are shown in grey.**

| **Gene name** | **Biological function and references** |
|---|---|
| Acetyl-Coenzyme A dehydrogenase, medium chain (Acadm) | |
| ARP2 actin-related protein 2 homolog (Actr2) | |
| Amyloid beta (A4) precursor protein (App) | |
| Annexin A2 (Anxa2) | Role in actin-assembly |
| Calmodulin 1 (Calm1) | |
| Caveolin, caveolae protein 1 Cav1) | Role in lipid homeostasis, |
| Cyclin G1 (Ccgn1) | |
| Cold shock domain containing E1 (Csde) | |
| Expressed sequence AW112037 | |
| Fibroblast growth factor 1 (Fgf1) | Regulator of human adipogenesis |
| Fibronectin type III domain containing 3B (Fndc3b) | Role in adipogenesis |
| Kinesin family member 5B (Kif5b) | Role in insulin-stimulated GLUT4 translocation to the plasma membrane |
| Mesoderm specific transcript (Mest) | Adipocyte differentiation and enlargement |
| Nucleosome assembly protein 1-like 1 (Nap1L1) | |
| Nidogen 1 (Nid1) | |
| natriuretic peptide receptor 3 (Npr3) | Possible role in sodium retention characteristic of obesity associated hypertension |
| nuclear undecaprenyl pyrophosphate synthase 1 homolog (Nus1) | |
| Phosphatidylinositol 3-kinase, catalytic, alpha polypeptide (Pik3ca) | Essential for proper growth factor signaling. Role in adipogenesis |

| **Placenta-specific 8 (Plac8)** | |
|---|---|
| Pleckstrin homology domain containing, family C (Plekhc1) | |
| Protein tyrosine phosphatase 4a1 (Ptp4a1) | Implicated in cell growth, differentiation, and tumor invasion |
| Related RAS viral (Rras2) oncogene homolog 2 | |
| Retinitis pigmentosa 9 homolog (Rp9h) | |
| Secreted acidic cysteine rich glycoprotein (Spare) | Mediates cell-matrix interactions and play a differentiation and angiogenesis |
| Signal-induced proliferation-associated 1 like 1 (Sipa1L1) | |
| Spectrin beta 2 (Spnb2) | |
| ST3 beta-galactoside alpha-2,3-sialyltransferase 6 (St3gal6) | |
| Vestigial like 3 (VgII3) | |
| Very low density lipoprotein receptor (Vldlr) | Involved in lipolysis |
| Zinc finger, DHHC domain containing 2 (Zdhhc2) | |
| WD repeat domain 26 (Wdr26) | |
| WW domain containing transcription regulator 1 (Wwtr1) | regulates mesenchymal stem cell differentiation |
| Expressed sequence AW112037 | |
| RIKEN cDNA B930093H17 gene (like-glycosyltransferase) | |

### Example 2: Tissue and cellular expression of the selected genes

To better understand the role of Plac8 in adipocytes development, its pattern of expression was first characterized. mRNA levels were measured by northern-blot and RT-PCR in various mouse tissues, in isolated preadipocytes and adipocytes, in visceral adipose tissue (VAT) and subcutaneous adipose tissue (SCAT) of mouse obesity model (Ob/Ob mice) and in human adipose tissues.

By northern-blotting, it was shown that Plac8 (1kb signal indicated by an arrow in figure 2A) is expressed at the same high level in SCAT and spleen of chow-diet C57BL/6J mice and at lower level in VAT, SCAT, muscle, heart, lung and muscle (Figure 2A). The expression patterns of Plac8 were then observed by microarray studies. In white adipose tissues of Ob/Ob mice, Plac8 level is decreased compared to level in wild type mice (Figure 2B). Values are expressed as fold increase relative to the control values in SCAT set arbitrarily at 1.

Adipose tissue is a complex tissue that includes not only mature adipocytes, but also precursor cells such as preadipocytes as well as blood vessels, macrophages and fibroblastic cells. Based on a collagenase I digestion technique, stromal vascular fraction (SVF) (including preadipocyte, endothelial and macrophage cells) was separated from the isolated adipocyte fraction. It was found that Plac8, is predominantly expressed in the stromal vascular fraction, containing preadipocytes (Figure 2C). These results indicate that Plac8, is more expressed in preadipocytes and thus appears to be involved in differentiation or proliferation processes.

The next step was to determine whether Plac8 gene is conserved among species. To address this question, a RT-PCR was performed on human adipose tissue samples. Preadipocytes and adipocytes were isolated from SCAT or VAT. Isolated preadipocytes were induced to differentiate *in vitro* until day 7. Results showed that Plac8 is indeed expressed in human fat (Figure 2D).They indicate that these genes are present in human adipose tissues. Altogether these results suggest that Plac8 is a relevant candidate gene for adipocytes development, possibly required for adipogenesis or fat tissue enlargement in obesity.

### Example 3: Expression of selected genes during 3T3-L 1 differentiation

Next, the expression of Plac8 gene was assessed during adipogenesis. For that purpose, mRNA levels were measured by RT-PCR during a detailed differentiation time-course of 3T3-L1 (an adipogenic cell line) (Figure 2E). The experiment showed that Plac8 is markedly increased in early step (1 to 3 hours after DMI treatment). This pattern is interesting since known adipogenic transcription factors such as CEBPr3 and γ (Rosen E.D. et *al*, 202), Krox20 (Chen, Z. et *al*., 2005) and Ebf (Jiminez, M.A. et *al*., 2007) show similar expression, suggesting the involvement of this gene in the early steps of adipogenesis.

### Example 4: shRNA knockdown of Plac8 in 3T3-L1 cells reduces adipogenesis

For the loss-of-function studies, shRNA specific for Plac8 subcloned into a retroviral vector from Clontech were used (RNAi-Ready pSIREN-RetroQ ZsGreen or pSIREN). This plasmid contains a GFP marker, which allows to control the infection efficiency in 3T3-L1 cells. Three different shRNA for Plac8, were cloned into the pSIREN plasmid, and were first tested in 293T HEK cells. This experiment demonstrated the ability of shRNA specific for Plac8 to inhibit Plac8 expression. Interestingly, 75% and 40% of knockdown were obtained with shPlac8-2 and shPlac8-3 respectively (Figure 3A), both of them being thus used for transduction into 3T3-L1 cells.

3T3-L1 cells were then infected for 6 hours with retroviral vectors expressing shRNA directed towards either Plac8 (shPlac8) or luciferase (shLuc). Using the GFP marker, we observed 90 % infection in the 3T3-L1 cells. At day 0, a 50 % knockdown for Plac8 was obtained in cells infected with both shPlac8-2 and shPlac8-3 (Figure 3B) whereas no inhibition was obtained with shLuc control. Then, cells were allowed to reach confluence and after one week differentiated with DMI. After 7 to 10 days of differentiation, cells were stained to determine the amount of lipid content with oil-red-O staining. Knockdown of Plac8 reduces adipogenesis as shown by the decrease of lipid staining and marker of adipogenesis in cells transfected with shPlac8 compared to control cells transfected with shLuc (Figures 3C and 3D).

### Example 5: Overexpression of Plac8 in 3T3-L1 cell line increase adipogenesis.

For the gain-of-function study, the cDNA of the murine sequence of Plac8 was subcloned into the pMSCV retroviral plasmid from Clontech. After infection of 3T3-I1 cells, RNA levels of Plac8 were measured by RT-PCR. At day 0, prior the differentiation, we obtained 3,5 fold induction of Plac8 in 3T3-I1 cells overexpressing Plac8 (L1 Plac8) compare to the control cells infected with the empty plasmid (L1 control) (Figure 4A). Cells were allowed to reach confluence and differentiated with DMI. At day 4 and day 9, cells were stained for lipid content with oil-red-O. As shown in Figure 4B, overexpression of Plac8 increases the adipogenic potential of 3T3-L1. A marker of differentiation (PPARg2) was also measured by RT-PCR, and the result showed that this marker was increased by 54 % in 3T3-L1 overexpressing Plac8 compare to control cells at day 9 (Figure 4C).

### Bibliography

Banerjee, S. S., M. W. Feinberg, M. Watanabe, S. Gray, R. L. Haspel, D. J. Denkinger, R. Kawahara, H. Hauner, and M. K. Jain. 2003. The Kruppel-like factor KLF2 inhibits peroxisome proliferator-activated receptor-gamma expression and adipogenesis. J Biol Chem. 278:2581-4. Epub 2002 Nov 7.
Chen, Z., J. I. Torrens, A. Anand, B. M. Spiegelman, and J. M. Friedman. 2005. Krox20 stimulates adipogenesis via C/EBPbeta-dependent and -independent mechanisms. Cell Metab. (2):93-106.
Collins, S., T. L. Martin, R. S. Surwit, and J. Robidoux. 2004. Genetic vulnerability to diet-induced obesity in the C57BL/6J mouse: physiological and molecular characteristics. Physiol Behav 81:243-8.
De Fourmestraux V, Neubauer H, Poussin C, Farmer P, Falquet L, Burcelin R, Delorenzi M and Thorens B, 2004 Transcript profiling suggests that differential metabolic adaptation of mice to a high fat diet is associated with changes in liver to muscle lipid fluxes. J. Biol. Chem279:50743-53
Gray, S., M. W. Feinberg, S. Hull, C. T. Kuo, M. Watanabe, S. Sen-Banerjee, A. DePina, R. Haspel, and M. K. Jain. 2002. The Kruppel-like factor KLF15 regulates the insulin-sensitive glucose transporter GLUT4. J Biol Chem 277:34322-8.
Jimenez, M. A., P. Akerblad, M. Sigvardsson, and E. D. Rosen. 2007. Critical role for Ebf1 and Ebf2 in the adipogenic transcriptional cascade. Mol Cell Biol 27:743-57.
Kang, S., C. N. Bennett, I. Gerin, L. A. Rapp, K. D. Hankenson, and O. A. Macdougald. 2007. Wnt signaling stimulates osteoblastogenesis of mesenchymal precursors by suppressing CCAAT/enhancer-binding protein alpha and peroxisome proliferator-activated receptor gamma. J Biol Chem 282:14515-24.
Ledford, J. G., M. Kovarova, and B. H. Koller. 2007. Impaired host defense in mice lacking ONZIN. J Immunol 178:5132-43.
Rogulski, K., Y. Li, K. Rothermund, L. Pu, S. Watkins, F. Yi, and E. V. Prochownik. 2005. Onzin, a c-Myc-repressed target, promotes survival and transformation by modulating the Akt-Mdm2-p53 pathway. Oncogene 24:7524-41.
Rosen, E. D. C. H. Hsu, X. Wang, S. Sakai, M. W. Freeman, F. J. Gonzalez, and B. M. Spiegelman. 2002. C/EBPalpha induces adipogenesis through PPARgamma: a unified pathway. Genes Dev 16:22-6.
Sahber et al., (1987), Biochem Int Bhattacharyya A, Murchie AI, Lilley DM. 1990. RNA bulges and the helical periodicity of double-stranded RNA. Nature. 1990 Feb 1;343 (6257):484-7.

## Claims

1. Inhibitor of the activity of Plac8 for the modulation of adipogenesis.

2. Inhibitor according to claim 1 which reduces adipogenesis.

3. Inhibitor according to claim 1 or 2 for the treatment of obesity and related disorders.

4. Inhibitor according to claim 1 or 2 for the reduction of visceral and/or subcutaneous fat accumulation

5. Inhibitor according to any previous claim wherein said inhibitor is a small molecule

6. Inhibitor according to any previous claim wherein said inhibitor is small interfering RNA

7. Inhibitor according to claim 7 wherein the siRNA is a shRNA having a sequence corresponding to SEQ ID NO.5 or SEQ ID NO.6 or SEQ ID NO.7.

8. Use of an inhibitor of the activity of Plac8 for the manufacture of a medicament for the modulation of adipogenesis.

9. Nucleic acid having sequence SEQ ID NO.6 or SEQ ID NO.7

10. Nucleic acid being a siRNA specific for Plac8 transcriptional inhibition

11. Method for screening for inhibitors of the activity of Plac8 comprising
a) transfecting a cell line with a reporter construction comprising a Plac8 promoter linked to a reporter gene
b) cultivating said cell line in condition to allow expression of the reporter gene
c) adding candidate compound into the cell culture, and
d) identifying inhibitor compounds as being those compounds which have the ability to reduce or inhibit the reporter gene expression

12. Composition comprising an inhibitor of Plac8 activity and at least one pharmaceutically acceptable excipient.

13. Composition according to claim 12 to treat obesity and related diseases.

14. Composition according to claim 12 for reduction of visceral and/or subcutaneous fat accumulation

15. Method of modulation of adipogenesis consisting in administration to a patient in need thereof of an inhibitor of Plac8 to modulate adipogenesis.


SEQUENCE LISTING

<110>  SANOFI-AVENTIS

<120>  Use of inhibitors of Plac8 activity for the modulation of
       adipogenesis

<130>  Plac8

<160>  23

<170>  PatentIn version 3.3

<210>  1
<211>  696
<212>  DNA
<213>  Mus musculus


<220>
<221>  CDS
<222>  (91)..(429)

<400>  1
ctatttgtag taagactcaa ccccagacca caggaccggt tctgcccaac ccttttgaac      60

tacttggtct tttgagacct cgcatcgaag atg gct cag gca cca aca gtt atc      114
                                 Met Ala Gln Ala Pro Thr Val Ile
                                 1               5

gtg act caa cct gga ttc gtt cgt gct ccc caa aat tcc aac tgg cag      162
Val Thr Gln Pro Gly Phe Val Arg Ala Pro Gln Asn Ser Asn Trp Gln
    10                  15                  20

acc agc ctg tgt gat tgc ttc agt gac tgc gga gtc tgc ctc tgt ggg      210
Thr Ser Leu Cys Asp Cys Phe Ser Asp Cys Gly Val Cys Leu Cys Gly
25                  30                  35                  40

acc ttt tgt ttc act tgt ctt gga tgt caa gtg gca gct gac atg aat      258
Thr Phe Cys Phe Thr Cys Leu Gly Cys Gln Val Ala Ala Asp Met Asn
                45                  50                  55

gag tgt tgt ctg tgt gga aca acg gtg gcc atg agg act ctc tac cga      306
Glu Cys Cys Leu Cys Gly Thr Thr Val Ala Met Arg Thr Leu Tyr Arg
            60                  65                  70

acc cga tac ggc att cct gga tct att tgt gat gac tac atg gtc aca      354
Thr Arg Tyr Gly Ile Pro Gly Ser Ile Cys Asp Asp Tyr Met Val Thr
        75                  80                  85

ctc ttc tgt cct gtt tgc tct gtg tgc caa ctc aag aga gac att aac      402
Leu Phe Cys Pro Val Cys Ser Val Cys Gln Leu Lys Arg Asp Ile Asn
    90                  95                  100

agg agg aga gcc atg aac gct ttc taa ggagctggat ggcaagagct            449
Arg Arg Arg Ala Met Asn Ala Phe
105                 110

ctggctgaag aagctcaact cagcacacac tccttcagcc tgagattttt caaatctttg      509

gcaactgaga tgggatggat ccatttaatt agagaacggt gaaatctttc tagttgggct      569

ttttgattta ttttaaatgg atattgctct ttgacttggt ttcttcttgc tcccatatca      629

tcaaatattg gagcctataa tttttttacc ttacatttta ggtagaaacc aaataaaaga      689

ttttgct                                                                696

<210>  2
<211>  112
<212>  PRT
<213>  Mus musculus

<400>  2

Met Ala Gln Ala Pro Thr Val Ile Val Thr Gln Pro Gly Phe Val Arg
1               5                   10                  15

Ala Pro Gln Asn Ser Asn Trp Gln Thr Ser Leu Cys Asp Cys Phe Ser
            20                  25                  30

Asp Cys Gly Val Cys Leu Cys Gly Thr Phe Cys Phe Thr Cys Leu Gly
        35                  40                  45

Cys Gln Val Ala Ala Asp Met Asn Glu Cys Cys Leu Cys Gly Thr Thr
    50                  55                  60

Val Ala Met Arg Thr Leu Tyr Arg Thr Arg Tyr Gly Ile Pro Gly Ser
65                  70                  75                  80

Ile Cys Asp Asp Tyr Met Val Thr Leu Phe Cys Pro Val Cys Ser Val
                85                  90                  95

Cys Gln Leu Lys Arg Asp Ile Asn Arg Arg Arg Ala Met Asn Ala Phe
            100                 105                 110

<210>  3
<211>  760
<212>  DNA
<213>  Homo sapiens

<220>
<221>  CDS
<222>  (102)..(449)

<400>  3
gagttttcat ttgtggtgag attctctccc aggccacaag acatttcctg ctcggaacct      60

tgtttactaa tttccactgc ttttaaggcc ctgcactgaa a atg caa gct cag gcg     116
                                              Met Gln Ala Gln Ala
                                              1               5

ccg gtg gtc gtt gtg acc caa cct gga gtc ggt ccc ggt ccg gcc ccc       164
Pro Val Val Val Val Thr Gln Pro Gly Val Gly Pro Gly Pro Ala Pro
                10                  15                  20

cag aac tcc aac tgg cag aca ggc atg tgt gac tgt ttc agc gac tgc       212
Gln Asn Ser Asn Trp Gln Thr Gly Met Cys Asp Cys Phe Ser Asp Cys
            25                  30                  35

gga gtc tgt ctc tgt ggc aca ttt tgt ttc ccg tgc ctt ggg tgt caa       260
Gly Val Cys Leu Cys Gly Thr Phe Cys Phe Pro Cys Leu Gly Cys Gln
        40                  45                  50

gtt gca gct gat atg aat gaa tgc tgt ctg tgt gga aca agc gtc gca       308
Val Ala Ala Asp Met Asn Glu Cys Cys Leu Cys Gly Thr Ser Val Ala
    55                  60                  65

atg agg act ctc tac agg acc cga tat ggc atc cct gga tct att tgt     356
Met Arg Thr Leu Tyr Arg Thr Arg Tyr Gly Ile Pro Gly Ser Ile Cys
70                  75                  80                  85

gat gac tat atg gca act ctt tgc tgt cct cat tgt act ctt tgc caa     404
Asp Asp Tyr Met Ala Thr Leu Cys Cys Pro His Cys Thr Leu Cys Gln
                90                  95                  100

atc aag aga gat atc aac aga agg aga gcc atg cgt act ttc taa         449
Ile Lys Arg Asp Ile Asn Arg Arg Arg Ala Met Arg Thr Phe
            105                 110                 115

aaactgatgg tgaaaagctc ttaccgaagc aacaaaattc agcagacacc tcttcagctt     509

gagttcttca ccatcttttg caactgaaat atgatggata tgcttaagta caactgatgg     569

catgaaaaaa atcaaatttt tgatttatta taaatgaatg ttgtccctga acttagctaa     629

atggtgcaac ttagtttctc cttgctttca tattatcgaa tttcctggct tataaacttt     689

ttaaattaca tttgaaatat aaaccaaatg aaatatttta actgataaaa aaaaaaaaaa     749

aaaataaaaa a                                                          760

<210>  4
<211>  115
<212>  PRT
<213>  Homo sapiens

<400>  4

Met Gln Ala Gln Ala Pro Val Val Val Val Thr Gln Pro Gly Val Gly
1               5                   10                  15

Pro Gly Pro Ala Pro Gln Asn Ser Asn Trp Gln Thr Gly Met Cys Asp
            20                  25                  30

Cys Phe Ser Asp Cys Gly Val Cys Leu Cys Gly Thr Phe Cys Phe Pro
        35                  40                  45

Cys Leu Gly Cys Gln Val Ala Ala Asp Met Asn Glu Cys Cys Leu Cys
    50                  55                  60

Gly Thr Ser Val Ala Met Arg Thr Leu Tyr Arg Thr Arg Tyr Gly Ile
65                  70                  75                  80

Pro Gly Ser Ile Cys Asp Asp Tyr Met Ala Thr Leu Cys Cys Pro His
                85                  90                  95

Cys Thr Leu Cys Gln Ile Lys Arg Asp Ile Asn Arg Arg Arg Ala Met
            100                 105                 110

Arg Thr Phe
        115

<210>  5
<211>  66
<212>  DNA

<213>  Artificial

<220>
<223>  scRNA


<220>
<221>  misc_RNA
<222>  (1)..(66)
<223>  shPlac8-1

<400>  5
gatccgtcgt gactcaacct ggattttcaa gagaaatcca ggttgagtca cgatttttta      60

cgcgtg                                                                  66


<210>  6
<211>  66
<212>  DNA
<213>  Artificial

<220>
<223>  scRNA


<220>
<221>  misc_RNA
<222>  (1)..(66)
<223>  shPlac8-2

<400>  6
gatccgctga catgaatgag tgttgttcaa gagacaacac tcattcatgt cagtttttta      60

cgcgtg                                                                  66


<210>  7
<211>  65
<212>  DNA
<213>  Artificial

<220>
<223>  scRNA


<220>
<221>  misc_RNA
<222>  (1)..(65)
<223>  shPlac8-3

<400>  7
gatccacggc attcctggat ctatttcaag agaatagatc caggaatgcc gtttttttac      60

gcgtg                                                                   65


<210>  8
<211>  63
<212>  DNA
<213>  Artificial

<220>
<223>  scRNA


<220>
<221>  misc_RNA

<222>  (1)..(63)
<223>  shLuc

<400>  8
gatccgtgcg ttgctagtac caattcaaga gattggtact agcaacgcac ttttttacgc     60

gtg                                                                   63


<210>  9
<211>  20
<212>  DNA
<213>  Artificial

<220>
<223>  Primer mPlac8-F

<400>  9
aaggagctgg atggcaagag                                                 20


<210>  10
<211>  23
<212>  DNA
<213>  Artificial

<220>
<223>  Primer mPlac8-R

<400>  10
ctgaaggagt gtgtgctgag ttg                                             23


<210>  11
<211>  19
<212>  DNA
<213>  Artificial

<220>
<223>  Primer mPPARgamma2-F

<400>  11
cagcgactgc ggagtctgt                                                  19


<210>  12
<211>  17
<212>  DNA
<213>  Artificial

<220>
<223>  Primer mPPARgamma2-R

<400>  12
acccaaggca cgggaaa                                                    17


<210>  13
<211>  19
<212>  DNA
<213>  Artificial

<220>
<223>  Primer mAp2-F

<400>  13
gcccaccaac ttcggaatc                                                  19

<210>  14
<211>  20
<212>  DNA
<213>  Artificial

<220>
<223>  Primer mAp2-R

<400>  14
tgcgagtggt cttccatcac                                                    20


<210>  15
<211>  19
<212>  DNA
<213>  Artificial

<220>
<223>  Primer mCyclophilinA-F

<400>  15
ccgcagacga caggaaggt                                                     19


<210>  16
<211>  15
<212>  DNA
<213>  Artificial

<220>
<223>  Primer mCyclophilinA-R

<400>  16
agggccccgc catct                                                         15


<210>  17
<211>  20
<212>  DNA
<213>  Artificial

<220>
<223>  Primer hPlac8-F

<400>  17
ttttgacttg cgggcatttt                                                    20


<210>  18
<211>  22
<212>  DNA
<213>  Artificial

<220>
<223>  Primer hPlac8-R

<400>  18
ggacgctctc ctgagctaca ga                                                 22


<210>  19
<211>  20
<212>  DNA
<213>  Artificial

<220>
<223>  Primer hCyclophilinA-F

<400>  19
ttcatctgca ctgccaagac                                                   20


<210>  20
<211>  20
<212>  DNA
<213>  Artificial

<220>
<223>  Primer hCyclophilinA-R

<400>  20
tcgagttgtc cacagtcagc                                                   20


<210>  21
<211>  637
<212>  DNA
<213>  Mus sp.


<220>
<221>  CDS
<222>  (1)..(336)

<400>  21
atg gct cag gca cca aca gtt atc gtg act caa cct gga ttc gtt cgt      48
Met Ala Gln Ala Pro Thr Val Ile Val Thr Gln Pro Gly Phe Val Arg
1               5                   10                  15

gct ccc caa aat tcc aac tgg cag acc agc ctg tgt gat tgc ttc agt      96
Ala Pro Gln Asn Ser Asn Trp Gln Thr Ser Leu Cys Asp Cys Phe Ser
            20                  25                  30

gac tgc gga gtc tgc ctc tgt ggg acc ttt tgt ttc act tgt ctt gga     144
Asp Cys Gly Val Cys Leu Cys Gly Thr Phe Cys Phe Thr Cys Leu Gly
        35                  40                  45

tgt caa gtg gca gct gac atg aat gag tgt tgt ctg tgt gga aca acg     192
Cys Gln Val Ala Ala Asp Met Asn Glu Cys Cys Leu Cys Gly Thr Thr
    50                  55                  60

gtg gcc atg agg act ctc tac cga acc cga tac ggc att cct gga tct     240
Val Ala Met Arg Thr Leu Tyr Arg Thr Arg Tyr Gly Ile Pro Gly Ser
65                  70                  75                  80

att tgt gat gac tac atg gtc aca ctc ttc tgt cct gtt tgc tct gtg     288
Ile Cys Asp Asp Tyr Met Val Thr Leu Phe Cys Pro Val Cys Ser Val
                85                  90                  95

tgc caa ctc aag aga gac att aac agg agg aga gcc atg aac gct ttc     336
Cys Gln Leu Lys Arg Asp Ile Asn Arg Arg Arg Ala Met Asn Ala Phe
            100                 105                 110

taaggagctg gatggcaaga gctctggctg aagaagctca actcagcaca cactccttca     396

gcctgagatt tttcaaatct ttggcaactg agatgggatg gatccattta attagagaac     456

ggtgaaatct ttctagttgg gctttttgat ttattttaaa tggatattgc tctttgactt     516

ggtttcttct tgctcccata tcatcaaata ttggagccta taattttttt accttacatt     576

ttaggtagaa accaaataaa agattttgct aagaagaaaa aaaaaaaaaa aaaaaaaaaa     636

a                                                                     637

<210> 22
<211> 112
<212> PRT
<213> Mus sp.

<400> 22

Met Ala Gln Ala Pro Thr Val Ile Val Thr Gln Pro Gly Phe Val Arg
1               5                   10                  15

Ala Pro Gln Asn Ser Asn Trp Gln Thr Ser Leu Cys Asp Cys Phe Ser
            20                  25                  30

Asp Cys Gly Val Cys Leu Cys Gly Thr Phe Cys Phe Thr Cys Leu Gly
        35                  40                  45

Cys Gln Val Ala Ala Asp Met Asn Glu Cys Cys Leu Cys Gly Thr Thr
    50                  55                  60

Val Ala Met Arg Thr Leu Tyr Arg Thr Arg Tyr Gly Ile Pro Gly Ser
65                  70                  75                  80

Ile Cys Asp Asp Tyr Met Val Thr Leu Phe Cys Pro Val Cys Ser Val
                85                  90                  95

Cys Gln Leu Lys Arg Asp Ile Asn Arg Arg Arg Ala Met Asn Ala Phe
            100                 105                 110

<210> 23
<211> 1000
<212> DNA
<213> Mus sp.

<220>
<221> promoter
<222> (1)..(1000)

<400> 23
catacataca tacatacata catacatact gatacagagg ctcataactg taatcccaga 60
actcctacga gagactgggt ggggggtgga ggcaggagaa ttgcttggaa gctcacagct 120
gtgcagcaca gtgggaacaa gagacaaggc agcttcaaca ggaggagaga agagacttcc 180
caaagctgtc ctcctgtccc ctgacctcca catgcctgct gtgatcctca gatgctgaca 240
tgtatgttca gacacacacc acagagagat ggggtgggag aaggtgatgg tgatgacaac 300
tacgacagat aaaaaataaa ataataaaaa tcacgcctaa cataaagcat aacataacat 360
aactaacata aagtgttcag tctgttacag aaaccaaagc aatatagcaa tattggggga 420
cagaggtagg tcaataatag agctctcacc taaaatgcac tggccctggg ttcagtccct 480
aatgcctcag ggaggaaaag aaaggggggg ggggaggaag aaaagacgga ggagaaagat 540
ctgatcagaa gcccggcatg gtggtgcatg tctttaatcc cagccacagg aggcagaggc 600
agatgggtct ctgagagttt gagaccatcc tacaaactga gctctgggat agccagaact 660

ctagagagac acactcaagg acagtctgag cgggcctgga actccaggtc cccaagtgcc 720
ttctggtttc ttaggttaaa agaggaaaat aaggtgtgag actcggagag ctttgtcagg 780
caggtagcta atcaggggaa ccacaccctc tcctttccac cgagacctta gaggttagcc 840
cttggaattg taaggaggaa aaccctattt ggtaagagat ggcttttggt gcctggatta 900
ccacagccaa tcagagcaca ggacattgct ctttgtactc cagcccaccc ctaccccacc 960
ctccacgggg ttgatacctc ctcctttcct cggagtctct 1000